# FASCICULE DE BREVET EUROPEEN

(11) **EP 0 406 404 B1**
(45) Date de publication et mention de la délivrance du brevet: **22.06.1994**
(21) Numéro de dépôt: 90902636.1
(22) Date de dépôt: 22.01.1990
(51) Int. Cl.: G01N 33/546, G01N 33/547

(54) **PARTICULES SUMICRONIQUES, PREPARATION ET UTILISATION DANS L'IMMUNODIAGNOSTIC**
SUBMIKRONISCHE TEILCHEN, HERSTELLUNG UND VERWENDUNG IN DER IMMUNDIAGNOSE
SUBMICRON PARTICLES, PREPARATION AND UTILIZATION IN IMMUNODIAGNOSIS

(30) Priorité: 20.01.1989 FR 8900660; 12.04.1989 FR 8904819
(43) Date de publication de la demande: 09.01.1991
(73) Titulaire: DIAGNOSTICA STAGO, F-92600 Asnières (FR)
(72) Inventeur: AMIRAL, Jean, F-95130 Franconville (FR); LAROCHE, Pascale, F-54000 Nancy (FR)
(74) Mandataire: Clisci, Serge
(86) Numéro de dépôt international: FR9000045
(87) Numéro de publication internationale: WO9008321

(56) Documents cités:
- EP-A- 0 295 402
- EP-A- 0 308 233
- FR-A- 2 125 000
- FR-A- 2 495 326
- US-A- 4 680 332

## Description

La présente invention concerne des particules de latex submicroniques (i.e. d'un diamètre moyen inférieur à 1 micromètre) sensibilisées par un matériel immunologique, c'est-à-dire des particules sur lesquelles sont fixés par covalence ou adsorption un antigène ou un anticorps. Ces particules sont "invisibles" ou "transparentes" quand elles sont exposées à un rayonnement électromagnétique, tel que la lumière visible et les UV, et deviennent "visibles" par agglutination quand elles sont exposées audit rayonnement après mise en contact avec un anticorps ou respectivement un antigène.

La présente invention concerne également le procédé de préparation de ces particules sensibilisées et leur utilisation dans les dosages immunologiques mettant en oeuvre des réactions du type antigène/anticorps.

### ART ANTERIEUR

Les techniques immunologiques ont pris une place importante au sein de l'analyse biomédicale. On les emploie aujourd'hui pour quantifier ou doser de nombreuses substances parmi lesquelles on peut notamment citer les protéines, les haptènes, les hormones, les médicaments et les anticorps.

Leur principe repose sur la fixation chimique d'un anticorps (ou d'un antigène) sur un support particulaire. Le complexe support particulaire/anticorps ou antigène constitue un réactif; ce réactif, mis en présence de l'antigène (ou de l'anticorps) correspondant agglutine rapidement. Cette agglutination s'accompagne d'une importante augmentation de l'absorbance du milieu, en fonction de la taille des particules. La particule constitue donc un élément amplificateur passif de l'agglutination se traduisant par une augmentation nette de l'absorbance.

Parmi les techniques connues, trois groupes de méthodes se sont imposés en immunoanalyse.

Le premier groupe comprend les méthodes de turbidimétrie et de néphélométrie. Il s'agit de méthodes de mise en oeuvre rapide, mais peu sensibles et uniquement utilisables pour les fortes concentrations protéiques, surtout pour la turbidimétrie qui implique des concentrations protéiques supérieures à 100 »g/ml. La néphélométrie est plus sensible (domaine d'utilisation de l'ordre de 20 »g/ml), mais requiert un matériel très "spécialisé" et un traitement préalable des échantillons qui restreignent fortement les possibilités d'utilisation.

Le second groupe comprend les méthodes dites de LAURELL et de MANCINI. Il s'agit de méthodes simples, mais de réalisation lente et non automatisable. Elles s'appliquent à des concentrations protéiques supérieures à 20 »g/ml pour la méthode de MANCINI et à 5 »g/ml pour la méthode de LAURELL. Les temps d'analyse sont de plusieurs heures et souvent de plus de 24 heures.

Le troisième groupe comprend les méthodes dites à marquage, notamment les méthodes immunoenzymologiques (EIA) et radioimmunologiques (RIA). Leur mise en oeuvre nécessite un appareillage coûteux ; les dosages comportent plusieurs étapes réactionnelles séparées par des lavages ; leur automatisation reste complexe et les temps d'analyse difficilement inférieurs à deux heures. Ces méthodes, par contre, permettent des sensibilités inférieures à 1 ng/ml et s'appliquent à toute substance biologique présente à des concentrations se situant avantageusement dans la gamme de 10 ng/ml à 1 mg/ml.

Cette situation justifie et explique les efforts de recherche entrepris aujourd'hui pour le développement de nouvelles méthodes d'immunodosage palliant les inconvénients précités en vue d'améliorer les conditions d'utilisation des techniques des deux premiers groupes lorsque la grande sensibilité des méthodes EIA et RIA n'est pas absolument nécessaire.

On sait en particulier que des auteurs se sont attachés depuis 1970 à affiner le mode de détection des réactions immunologiques de type antigène/anticorps des deux premiers groupes précités. Ainsi a été envisagée l'utilisation de microparticules de latex revêtues d'un antigène ou d'un anticorps pour la mise au point de dosages quantitatifs par turbidimétrie, par néphélométrie et par comptage de particules.

Dans le domaine de la turbidimétrie (par mesure de l'absorbance de la lumière) on connaît des articles de A.M. BERNARD, A. VYSKOCLL et R.R. LAUWERYS, Clin. Chem., 27 (No 6), pages 832-837, (1981) et de A.M. BERNARD et R.R. LAUWERYS, Clin. Chim. Acta, 119, pages 335-339, (1982), l'utilisation de particules submicroniques de latex (diamètre moyen : 0,79 micromètre) revêtues d'anticorps anti β₂-microglobuline pour le dosage de β₂-microglobuline par mesure de l'absorbance à 360 nm; de l'article de Y. MAYNARD et al., Clin. Chem., 32 (No 5), pages 752-757, (1986), l'utilisation d'anticorps monoclonaux ou polyclonaux anti-IgG pour le dosage, dans des microcuvettes d'immunoglobulines sériques.

Dans le domaine de la néphélométrie (par mesure de la diffusion de la lumière) on connait de l'article de J. GRANGE et al., Journal of Immunological Methods, 18, pages 365-375, (1977) l'utilisation de billes de latex submicroniques (diamètre moyen 0,3 micromètre) revêtues d'un anticorps, anti-IgG de lapin, pour le dosage d'IgG de lapin. Les billes de latex sont constituées par du polystyrène carboxylé contenant les groupes COOH pour fixer le ligand, dans le cas d'espèce l'anticorps anti-IgG de lapin. Cet article signale notamment que "la préparation de sphères de latex contenant des antigènes ou des anticorps adsorbés est difficile à standardiser en raison de leur tendance à s'autoagglutiner lorsque le pH et la force ionique du milieu de dosage changent".

On connaît également de l'article de Anne-Marie BONNEFOY et al., C.R. Acad. Sc. Paris, 283, série D pages 115-118 (5 juillet 1976) l'utilisation de sphères de latex (polystyrène) d'un diamètre de 300 nm liées par covalence à un anticorps pour la réalisation de dosages néphélométriques (lecture sous une longueur d'onde de 550 nm, selon un angle d'observation de 90°). Cet article ne décrit ni ne suggère le mode de stabilisation du réactif immunologique constitué par lesdites sphères de latex liées par covalence à l'anticorps et stabilisées.

Dans le domaine du comptage des particules on connaît l'utilisation de particules de polystyrène submicroniques (diamètre moyen : 0,8 micromètre) revêtues d'IgG humaine pour apprécier l'agglutination en présence d'un moyen agglutinant, tel que le Facteur rhumatoïde, de l'article de C.L. CAMBIASO et P.L. MASSON "automated determination of circulating immune complexes by particules counting immunoassay (PACIA)", Protides and Biological Fluids, 1-4, (1979); on connaît également les deux articles précités de A.M. BERNARD et al.; et, l'exposé de J.C. MARESCHAL intitulé "Une nouvelle génération de dosages immunologiques, non-isotopiques par comptage de particules de latex" présenté au cours des XXXèmes journées nationales de l'APDILA tenues à Paris les 16 et 17 novembre 1985, qui rappelle les difficultés rencontrées au cours des mesures impliquant l'agglutination des latex, telles que la difficulté de quantifier la réaction d'agglutination, la fréquence élevée d'agglutination non spécifique et le manque de sensibilité.

En bref, les méthodes turbidimétriques, néphélométriques et de comptage de particules sont limitées par plusieurs inconvénients à savoir :
- difficultés à réaliser des suspensions stables et standardisées,
- autoagglutination spontanée des latex sensibilisés par un anticorps ou un antigène,
- faible plage de mesure de la courbe d'étalonnage (variation de la densité optique (DO) n'excédant pas 0.3 à 0.4),
- manque de sensibilité et de reproductibilité,
- traitement du réactif particulaire avant emploi, dans certains cas (pour désaggrégation), et mauvaise stabilité du réactif,
- mise en oeuvre délicate et détection parfois complexe.
Ces inconvénients ont fait que ces méthodes n'ont pas été exploitées malgré leur intérêt pratique.

On connaît par ailleurs de l'article de J. WINKLES et al., "Enhanced-Latex-Agglutination Assay for C-Reactive Protein in Serum, with Use of a Centrifugel Analyzer", Clin. Chem., 33 (No 5), pages 685-689, (1987), l'utilisation de particules sphériques submicroniques de latex de polystyrène (diamètre moyen : 0,057 micromètre) sur lesquelles a été adsorbé un anticorps, dans le cas d'espèce un anticorps anti-Protéine C réactive, pour apprécier par comptage de particules (de préférence), turbidimétrie ou néphélométrie l'agglutination résultant de la mise en contact du complexe sphères de latex/anti-Protéine C réactive avec la Protéine C réactive (en abrégé : CRP). La technique décrite par J. WINKLES et al. implique un traitement par ultrasons (en anglais : "sonication") jugé essentiel pour disperser et stabiliser les particules de latex (pour empêcher leur autoagglutination) et donc de permettre l'utilisation de ces particules pour adsorber le matériel immunologique (dans le cas d'espèce dudit article l'anticorps anti-CRP). Le traitement par ultrasons, qui doit intervenir à nouveau avant toute utilisation du réactif immunologique constitué par le matériel immunologique adsorbé sur lesdites particules de latex, exige l'utilisation d'un matériel onéreux (à savoir le générateur d'ultrasons et son enceinte) et la mise en oeuvre de modalités opératoires compliquées perturbant ou limitant les possibilités de reproduction de ladite technique. Voir à cet effet les indications données dans ledit article page 686, colonne de gauche lignes 37-58, et, page 688, colonne de droite, lignes 54-62.

En bref, la technique de J. WINKLES et al., pour les dosages immunologiques du type antigène/anticorps exige la mise en oeuvre d'un champ d'ultrasons pendant une durée de 60 s; si cette durée est supérieure à 60 s, elle entraîne une surchauffe détruisant le complexe sphères de latex/anticorps, et si le traitement par ultrasons est incomplet, la sensibilité du dosage avec le complexe sphères de latex/anticorps résultant diminue considérablement et on est conduit à des résultats de dosage aberrants.

On sait aussi que le latex utilisé pour fixer, sous forme de sphères, de membranes ou de parois, des antigènes ou des anticorps doit être stabilisé notamment au moyen d'une substance polyhydroxylée ou protéinique ou encore de la polyvinylpyrrolidone (voir notamment EP-A-0 104 101, FR-B-2 125 000, FR-A-2 495 326, EP-A-0 140 489 EP-A-0 280 560, EP-A-0 163 393 et EP-A-0 281 327).

Il se trouve que le choix du latex est particulièrement important. On a en effet constaté que les particules submicroniques de latex notamment selon EP-A-0 296 883 (latex carboxypolypropylène), EP-A-0 286 687 (latex copolymères éthylène-acide acrylique et éthylène-acide méthacrylique), EP-A-0 295 402 (latex copolymère styrène-acide acrylique-méthacrylate de triéthylèneglycol), FR-B-2 125 000 (latex polystyrène ou polyacrylamide et latex copolymère butadiène-styrène, acrylonitrile-butadiène-styrène ou acétate de vinyle-acrylate de vinyle), FR-A-2 495 326, EP-A-0 140 489 et EP-A-0 280 560 (latex polystyrène, polyacrylamide, polyéthylène ou polypropylène), même associées à un matériau stabilisant, ne sont pas stables dans le temps. Ces particules sensibilisées par un matériel immunologique tel que antigène ou anticorps sont sujettes à des phénomènes d'auto-agglutination après 2-4 mois de stockage dans les conditions usuelles.

Pour avoir une amélioration de la stabilité lors du stockage à 4°C, il faudrait plutôt faire appel à un réactif immunologique dilué.

### BUT DE L'INVENTION

Il existe un besoin d'une technique de dosage immunologique mettant en oeuvre des antigènes ou anticorps liés par covalence ou adsorption à des particules de latex submicroniques, relativement simple, différente des techniques EIA et RIA, permettant de pallier les inconvénients précités de l'art antérieur, assurant une détermination rapide facilement automatisable et accessible à tout laboratoire d'analyse ou de recherche avec un vaste champ d'application, permettant d'atteindre un seuil de sensibilité d'au moins 100 ng/ml.

Il existe aussi un besoin d'une technique de préparation de particules submicroniques de latex sensibilisées par une substance ou matériel immunologique qui soient stables dans le temps lors du stockage sans que l'on soit obligé de les diluer dans un tampon biologique approprié.

Selon l'invention on se propose de satisfaire les besoins précités en fournissant une nouvelle solution technique qui fait appel à des particules submicroniques de latex particulier, d'une part et pallie les inconvénients de l'art antérieur rappelés ci-dessus, d'autre part.

### OBJET DE L'INVENTION

Selon l'invention on préconise une nouvelle solution technique pour la préparation de particules de latex sensibilisées par un matériel immunologique dans l'optique de réaliser des dosages immunologiques du type antigène/anticorps par agglutination de particules de latex revêtues par adsorption d'un antigène (ou d'un anticorps) lorsque lesdites particules sont mises en contact avec l'anticorps (ou respectivement l'antigène) correspondant.

Cette nouvelle solution technique comprend, d'une part, le choix d'un latex particulier, et d'autre part, le blocage ou l'occupation des sites actifs des particules, qui ne sont pas occupés par le matériel immunologique antigène ou anticorps, par un matériau stabilisant choisi parmi l'ensemble constitué par les substances hydroxylées contenant un ou mieux plusieurs groupes OH par molécule, les substances protéiniques, les substances peptidiques, les aminoacides, les substances polyosiques comportant un ou plusieurs groupes acide carboxylique et/ou carboxylate, la polyvinylpyrrolidone, leurs analogues et leurs mélanges.

Suivant un premier aspect de l'invention on préconise un procédé de fixation par covalence ou adsorption d'un matériel immunologique choisi parmi les antigènes et les anticorps sur des particules submicroniques de latex, ledit procédé, qui comporte la stabilisation du latex, étant caractérisé en ce qu'il comprend
(A) la mise en contact dans un milieu liquide approprié ayant une force ionique comprise dans la gamme de 0,01 à 0,5 à un pH de 4 à 10, à une température de 0 à 60°C
   (1) de particules de latex acrylique à base de polyméthacrylate de butyle, ayant un diamètre moyen inférieur ou égal à 100 nm, ledit latex étant un copolymère constitué de motifs styrène et de motifs méthacrylate de butyle dans un rapport molaire motifs styrène/motifs méthacrylate de butyle de 40/60 à 45/55 ayant une densité de 0,9 à 1,4 g/cm³ et comportant de 100 à 450 micro-équivalents de groupes COOH par gramme de latex, avec
   (2) le matériel immunologique
      pour obtenir des particules de latex sensibilisées par un matériel immunologique et telles que ledit matériel immunologique soit lié à chaque particule de latex, et
(B) la stabilisation dudit réactif immunologique ainsi obtenu au moyen d'un matériau stabilisant choisi parmi l'ensemble constitué par les substances hydroxylées contenant un ou mieux plusieurs groupes OH par molécule, les substances protéiniques, les substances peptidiques, les aminoacides, les substances polyosiques comportant un ou plusieurs groupes acide carboxylique et/ou carboxylate, la polyvinylpyrrolidone et leurs mélanges.

Suivant un second aspect de l'invention, on préconise en tant que produit industriel nouveau le réactif immunologique qui est constitué par lesdites particules revêtues par ledit matériel immunologique adsorbé et ledit matériau stabilisant, et qui est obtenu selon le procédé sus-visé, les dimensions dudit réactif immunologique étant telles que, lors de l'analyse sous un rayonnement ayant une longeur d'onde donnée (λ), le rapport longeur d'onde (λ) / diamètre moyen (d) desdites particules soit supérieur ou égal à 5/1.

Plus particulièrement selon l'invention lesdites particules de latex seront telles que:
(i) lesdites particules revêtues de ladite substance immunologique et dudit matériau stabilisant mises en suspension dans un milieu liquide aqueux sont invisibles quand elles sont exposées à un rayonnement avant une longueur d'onde (λ) supérieure au diamètre moyen (d) desdites particules, et
(ii) lesdites particules revêtues de ladite substance immunologique et dudit matériau stabilisant mises en suspension dans ledit milieu liquide aqueux deviennent "visibles" par agglutination quand elles sont exposées audit rayonnement après mise en contact avec une substance choisie parmi les anticorps et respectivement les antigènes et qui correspond à ladite substance immunologique fixée sur lesdites particules.

Suivant un troisième aspect de l'invention, on préconise l'utilisation de ce réactif immunologique dans le domaine des dosages immunologiques du type antigène/anticorps.

Les procédés de dosage suivant l'invention comprennent
- la détermination directe d'un anticorps par agglutination après mise en contact audit anticorps avec un réactif constitué par des particules de latex liées chacune par covalence ou adsorption à un antigène et stabilisées;
- la détermination directe d'un antigène par agglutination après mise en contact dudit antigène avec un réactif constitué par des particules de latex liées chacune par covalence ou adsorption à un anticorps; et,
- l'inhibition de l'agglutination du réactif latex/antigène en présence de l'anticorps correspondant et dosage indirect de l'antigène libre (notamment par compétition) ou vice-versa.

Ces procédés de dosage sont insensibles à la température dans les conditions usuelles d'utilisation et peuvent être mis en oeuvre à une température de 0 à 60°C, en particulier de 4 à 60°C et notamment à une température usuelle dans le domaine immunologique située dans la gamme de 15 à 37°C.

La technique de dosage suivant l'invention, qui repose avantageusement sur le changement invisibilité/visibilité de particules submicroniques par agglutination, se distingue de l'enseignement des solutions techniques de l'art antérieur sus-visé.

### DESCRIPTION DETAILLEE DE L'INVENTION

Suivant l'invention par les expressions "particules invisibles" et "particules visibles" on entend le fait que, d'une part, les particules dites invisibles ou transparentes n'absorbent ni ne diffusent le rayonnement électromagnétique, tel que la lumière, traversant le milieu contenant lesdites particules sans déviation substantielle dudit rayonnement, et d'autre part, les particules dites visibles absorbent et/ou diffusent ledit rayonnement électromagnétique.

Par l'expression "substance immunologique" ou "matériel immunologique" on entend toute substance choisie parmi l'ensemble constitué par les antigènes et les anticorps et devant être fixée par covalence ou adsorption sur les microparticules.

Par le terme "ligand" on entend ici l'antigène ou l'anticorps lié par covalence ou adsorption à chaque particule de latex.

Par l'expression "réactif immunologique" on entend l'ensemble constitué par les particules de latex et la substance immunologique choisie parmi les anticorps et respectivement les antigènes qui est liée par covalence ou adsorption à chacune desdites particules, lesdites particules de latex revétues dudit matériel immunologique étant stabilisées au moyen d'un matériau stabilisant.

Par les termes "substance" ou "composé correspondant" (homologue, complémentaire et/ou conjugué) d'un anticorps (ou d'un antigène), on entend l'antigène, ou respectivement l'anticorps, qui réagit avec l'anticorps, ou respectivement l'antigène, du réactif immunologique.

Le terme "antigène" désigne ici tout antigène proprement dit mais également toute substance à partir de laquelle on est susceptible de générer un anticorps; parmi les substances pouvant générer des anticorps on peut notamment citer les haptènes, les peptides, les médicaments comportant au moins un fragment peptidique, les alcaloïdes et d'une manière générale toute substance présentant une structure immunologique.

Comme indiqué plus haut le choix du latex est important pour la mise en oeuvre du procédé de fixation et du procédé de dosage selon l'invention. On préconise ici de faire appel à des particules de latex acrylique à base de polyméthacrylate de butyle, ayant un diamètre moyen inférieur ou égal à 100 nm, ledit latex étant un copolymère constitué de motifs styrène et de motifs méthacrylate de butyle dans un rapport molaire motifs styrène/motifs méthacrylate de butyle de 40/60 à 45/55, ayant une densité de 0,9 à 1,4 g/cm³ et comportant de 100 à 450 micro-équivalents de groupes COOH par gramme de latex.

De façon avantageuse, on recommande d'utiliser un copolymère constitué de motifs styrène et de motifs méthacrylate de butyle dans un rapport molaire motifs styrène/motifs méthacrylate de butyle de 42/58 à 43/57 ayant une densité de 1,0 à 1,1 g/cm³ et contenant de 250 à 300 micro-équivalents de groupes COOH par gramme de latex.

Parmi les particules de latex, qui conviennent selon l'invention, on préfère celles ayant un diamètre (d) de 67 nm, une densité de 1,0 à 1,1 g/cm³ et qui sont constituées d'un copolymère styrène-méthacrylate de butyle fabriqué par la société dite RHONE-POULENC dans lequel le rapport molaire motifs styrène/motifs méthacrylate de butyle est de 42,4/57,6 et qui comporte de 250 à 300 micro-équivalents de groupes COOH par gramme de latex.

Comme indiqué ci-dessus, il est essentiel que les particules de latex utiles selon l'invention aient un diamètre moyen (d) inférieur ou égal à 100 nm, de préférence compris entre 1 et 100 nm, et mieux situé dans la gamme allant de 5 à 100 nm.

Le rayonnement, électromagnétique utilisé selon l'invention pour apprécier la variation de l'agglutination lors de la réaction du réactif immunologique avec son conjugué doit alors avoir une longueur d'onde (λ) nettement supérieure au diamètre moyen (d) des particules de latex. De façon avantageuse ladite longueur d'onde sera située dans la gamme allant de 300 à 1000 nm (notamment de 400 à 700nm). Comme indiqué plus haut le rapport λ/d sera supérieur ou égal à 5.

Il est également important que le latex présente une fonctionnalité réactive avec la substance immunologique. Conviennent ici les latex comprenant une fonctionnalité COOH, c'est-à-dire des groupes COOH proprement dits ou, selon le pH du milieu, des groupes COO⁻ ou COOR' où R' est un groupe usuel de blocage dans le domaine immunologique pouvant notamment représenter un groupe alkyle en C₁-C₄, phényle ou benzyle, les groupes R' préférés étant ici les groupes alkyle en C₁-C₄ tel que méthyle, éthyle, isopropyle, t-butyle ou s-butyle.

En pratique les groupes réactifs de la fonctionnalité réactive avec la substance immunologique représenteront approximativement 100 à 450 micro-équivalents (dénommés ici groupes COOH par commodité) par gramme de latex.

Selon l'invention la fixation du matériel immunologique sur les particules de latex submicroniques est réalisée par covalence ou adsorption. La fixation par covalence se distingue de la fixation par adsorption par le fait que la fonctionnalité COOH, telle que définie plus haut, est dans le cas de la fixation par covalence activée ou sensibilisée par un composé carbodiimide ( qui est avantageusement soluble dans le milieu biologique de préparation).

Pour la fixation par covalence, la mise en contact du point (A) est réalisée selon un procédé qui est caractérisé en ce qu'il comprend la mise en contact pendant au moins 10 minutes dans un milieu aqueux tamponné à un pH de 4-10 et ayant une force ionique de 0,01 à 0,5 dudit matériel immunologique avec lesdites particules de latex dans lesquelles les sites actifs COOH ont été préalablement sensibilisés au moyen d'un composé carbodiimide puis l'addition d'un matériau stabilisant en excès pour bloquer les sites actifs encore libres sur lesdites particules de latex.

En variante la fixation par covalence peut comprendre, avant ladite mise en contact, la réaction des sites actifs desdites particules de latex (préalablement activés ou sensibilisés par réaction avec ledit composé carbodiimide) avec un composé bifonctionnel extendeur de chaîne dans un milieu aqueux tamponné à pH 4-10 et ayant une force ionique de 0,01 à 0,5 à une température de 40-60°C pendant au moins 10 minutes.

Ainsi les latex à fonctionnalité réactive COOH selon l'invention sont utilisables soit directement pour couplage covalent du ligand par réaction chimique soit après extension latérale de chaîne au moyen d'un bras bifonctionnel. Parmi les composés bifonctionnels utiles pour la réalisation du bras ou pont bifonctionnel qui conviennent selon l'invention on peut mentionner les diamines et les aminoacides comprenant un groupe amino en position terminale par rapport au groupe carboxylique, comme par exemple l'acide ε-aminocaproïque; ce bras ou pont est ensuite activé pour le couplage covalent.

La particularité majeure des latex est leur faculté à former des suspensions stables pouvant être déstabilisées de façon spécifique dans des conditions définies, dans le cas d'espèce la réaction antigène/anticorps. Suivant l'invention les particules sensibilisées par un antigène ou un anticorps, par exemple un haptène, conservent les propriétés inhérentes au latex et celle de la substance immunologique couplée. Lesdites particules restent monodispersées durant le stockage et s'agrègent uniquement en présence de l'élément complémentaire du système ligand/anti-ligand.

Eu égard à la fonctionnalité réactive la présence de groupements fonctionnels tels que COOH ou autres précités, à la surface des particules de latex permet un couplage covalent avec les groupements fonctionnels du ligand. De façon non limitative les couples fonctionnels COOH/NH₂ seront avantageusement utilisés selon l'invention. Bien entendu d'autres combinaisons de couples fonctionnels connus dans la technique sont également envisageables.

Des exemples non limitatifs de couplage par covalence sont donnés ci-après pour information :
- Activation des groupements COOH du latex par un carbodiimide hydrosoluble (par exemple, l'EDAC ou le morphocarbodiimide ou tout autre carbodiimide soluble) réagissant avec les groupements NH₂ du ligand à un pH compris entre 4 et 10 dans un milieu dépourvu de grou-groupements aminés libres; l'activation peut se faire en des temps avec élimination de l'excès de réactif de couplage (méthode préférentielle) ou directement en un temps; le ligand est ensuite ajouté en proportion adéquate, puis le latex est saturé et stabilisé.
- Activation des groupements COOH du latex par un carbodiimide et greffage d'un bras intermédiaire : les latex activés par le carbodiimide sont, dans un premier temps, "dérivatisés" à l'aide d'une diamine en excès (dans cette technique, on utilise habituellement une diamine aliphatique comprenant de 2 à 10 atomes de carbonne) afin d'éloigner le ligand de la surface, le rendant plus réactif, et de créer des groupements NH₂; les particules ainsi obtenues et dites "dérivatisées" sont ensuite activées par le glutaraldéhyde (dont l'excès est éliminé) ou le carbodiimide; après activation, elles réagissent avec les groupes NH₂ ou COOH du ligand.

L'utilisation de latex à fonctionnalité réactive comportant d'autres groupements réactifs confère des possibilités intéressanres de couplage covalent :
- latex carboxylé (COOH) activé par un carbodiimide ou un 2-éthoxy-1-(2H)-quinoléinecarboxylate, notamment le 2-éthoxy-1-(2H)-quinoléinecarboxylate d'éthyle (EEDQ),
- latex à groupements ester (COOR") activé par le glutaraldéhyde.

Pour la fixation par covalence ou par adsorption, la stabilisation du point (B) est réalisée pendant ou après la mise en contact du point (A). En d'autres termes, le matériau stabilisant prévu au point (B) peut être introduit dans le milieu biologique réactionnel lors de la mise en contact (A) ou après celle-ci. D'une façon générale, on préfère que ledit matériau stabilisant soit introduit après que ladite mise en contact ait été effectuée; dans le cas de la fixation par covalence, on peut introduire ledit matériau stabilisant dans ledit milieu de mise en contact à condition que ledit matériau stabilisant ne comporte pas de groupes réactifs NH₂.

Pour compléter la stabilisation, l'on peut encore introduire après l'addition du matériau stabilisant un ballast constitué d'un matériau protéinique ou polyhydroxylé, qui est particulièrement utile pour lyophiliser le réactif immunologique selon l'invention.

En variante on peut utiliser un tel ballast avant le point (A), pour la fixation par covalence du matériel immunologique sur les particules de latex, afin de former une cache moléculaire de liaison (albumine ou polymère organique tel que la polylysine) entre le latex et le ligand.

Dans le mode de sensibilisation des particules de latex et de fixation du ligand par covalence ou adsorption, les conditions expérimentales, à savoir le pH, la force ionique, le volume, la température, la durée et la concentration du ligand sont ajustées en fonction de la nature du produit à doser de façon à assurer :
- une parfaite stabilité du réactif immunologique particulaire dans un milieu biologique approprié,
- une réactivité maximale et une reproductibilité efficace, et
- une excellente conservation du réactif immunologique au stockage (on a observé à la fin d'une durée de conservation de 12-18 mois à + 4°C aucune dégradation ou inactivation substantielle dudit réactif).

D'un point de vue pratique, on constate que, quel que soit le tampon, l'augmentation du pH et de la force ionique entraîne une stabilisation du réactif immunologique, d'une part, et provoque une diminution proportionnelle de la réactivité, d'autre part.

C'est pourquoi on préconise pour la préparation du réactif immunologique un ajustement global, qui permet de pallier l'aspect antinomique sus-visé, et qui fait appel à un milieu aqueux liquide comprenant de l'eau et un moyen tampon, ayant un pH dans la gamme allant de 4 à 10, (avantageusement 6-9 et mieux 8,2-8,5), ayant une force ionique située dans la gamme de 0,01 à 0,5, et contenant, le cas échéant, pour stabiliser les particules de latex, un ballast inerte protéinique ou polyhydroxylé incorporé après la stabilisation du point (B).

De façon pratique, la réaction du réactif immunologique avec la substance complémentaire ou conjuguée sera effectuée dans un milieu liquide aqueux ayant un pH de 4-10, notamment un pH de 5-9, une force ionique de 0,01-0,5 et contenant le cas échéant un alcool polymère et/ou une substance choisie parmi les protéines et les aminoacides inertes.

Le procédé de dosage que l'on préconise suivant l'inven tion, qui met en oeuvre une réaction du type antigène/anticorps est caractérisé en ce qu'il consiste
(1) à mettre en contact, dans un milieu liquide aqueux ayant un pH situé dans la gamme de 4 à 10 et une force ionique située dans la gamme de 0,01 à 0,5, (i) des particules de latex, ledit latex étant un copolymère constitué de motifs styrène et de motifs méthacrylate de butyle dans un rapport molaire motifs styrène/motifs méthacrylate de butyle de 40/60 à 45/55, renfermant une quantité molaire de motifs méthacrylate de butyle supérieure à 50 %, ayant une densité de 0,9 à 1,4 g/cm³ et comportant de 100 à 450 microéquivalents de groupes COOH par gramme de latex, et lesdites particules ayant un diamêtre moyen (d) inférieur ou égal à 100 nm et étant revêtues d'une substance immunologique choisie parmi les antigènes et les anticorps et liée par covalence ou adsorption à chacune desdites particules, les sites actifs de chacune desdites particules non liés à ladite substance immunologique étant essentiellement bloqués par un matériau stabilisant choisi parmi l'ensemble constitué par les substances hydroxylées contenant un ou mieux plusieurs groupes OH par molécule, les substances protéiniques, les substances peptidiques, les aminoacides, les substances polyosiques comportant un ou plusieurs groupes acide carboxylique et/ou carboxylate, la polyvinylpyrrolidone, leurs analogues et leurs mélanges, avec (ii) une substance complémentaire ou conjuguée de ladite substance immunologique, et
(2) à apprécier l'évolution de l'agglutination par exposition à un rayonnement ayant une longueur d'onde (λ) située dans la gamme de 300 à 1000 nm (notamment 400-700 nm), telle que le rapport λ/d soit supérieur ou égal à 5.

Dans ce procédé, on apprécie soit l'agglutination propement dite soit l'inhibition de l'agglutination.

La température que l'on préconise pour la mise en oeuvre de ce procédé est située dans la gamme de 4 à 60°C et mieux de 15 à 37°C.

De préférence, le dosage sera réalisé par turbidimétrie. Le dosage par turbidimétrie offre l'avantage de permettre une utilisation aisée par tout laboratoire équipé uniquement d'un photomètre ou d'un spectrophotomètre (travaillant à une longueur d'onde comprise entre 300 et 1000 nm) ou équipé d'un appareillage automatique et sophistiqué (type analyseur centrifuge tel que l'analyseur COBAS Bio^{R}).

Le dosage peut également être réalisé par néphélométrie ou par comptage de particules avec, en contre-partie, une utilisation plus restrictive, compte tenu du coût de l'appareillage.

La sensibilité du dosage suivit l'invention est d'une manière générale d'au moins 100 ng/ml. Dans certains cas, le seuil de sensibilité, notamment par néphélométrie, pourra être inférieur afin d'être égal à au moins 10 ng/ml.

Selon un mode avantageux de mise en oeuvre conféré par l'invention, la durée du dosage sera en générale courte et notamment comprise entre 30 et 600 s.

Un tel dosage est applicable à la détermination quantitative d'antigènes et d'anticorps d'une part, de toutes substances susceptibles de générer des anticorps telles que les haptènes, les peptides, les médicaments comportant au moins un fragment peptide, et les alcaloïdes, d'autre part, dans divers milieux biologiques, tels que plasma, sérum, urine, liquide céphalorachidien, et extraits biologiques, pour divers secteurs de la technique tels que la santé animale, la santé humaine, le domaine agro-alimentaire, l'environnement et analogues.

Le meilleur mode de mise en oeuvre de l'invention consiste à effectuer la mise en contact du point (A) dans un milieu biologique aqueux ayant un pH de 6-9, et mieux de 8,2-8,5 (en particulier dans un tampon glycine de pH 8,35) et ayant une force ionique de 0,01-0,5. La durée de mise en contact du point (A) est d'au moins 10 minutes pour la fraction du matériel immunologique par covalence et d'au moins 60 minutes pour la fixation dudit matériel immunologique par adsorption.

Suivant ce meilleur mode de mise en oeuvre, l'on met en contact selon le point (A) 100 parties en poids de particules de latex avec 1 à 50 et de préférence 2 à 20 parties en poids de matériel immunologique, puis l'on utilise 10 à 100 parties en poids de matériau stabilisant pour 100 parties en poids de particules de latex utilisées lors de ladite mise en contact.

Suivant toujours ce meilleur mode, l'addition du moyen stabilisant intervient après la mise en contact du point (A) dans un milieu biologique identique ou similaire de celui de ladite mise en contact.

Pour la mise en oeuvre du procédé de dosage, on recommande de faire appel à un milieu biologique aqueux identique ou similaire à celui de la mise en contact du point (A).

D'autres avantages et caractéristiques de l'invention seront mieux compris à la lecture qui va suivre d'exemples de réalisation nullement limitatifs mais donnés à titre d'illustration.

Dans ces exemples, les exemples 1-4 ont trait à un réactif immunologique dans lequel le matériel immunologique est fixé par covalence aux particules submicroniques de latex, et les exemples 5-8 ont trait à un réactif immunologique dans lequel le matériel immunologique est fixé par adsorption aux particules submicroniques de latex.

### EXEMPLE 1

### Préparation du réactif immunologique

On fait appel à un latex à base de poly(méthacrylate de butyle), et plus précisément d'un copolymère styrène-méthacrylate de butyle où le rapport molaire motifs styrène/motifs méthacrylate de butyle est de 42,4/57,6 ayant une densité de 1,02 g/cm³, sous forme de billes ayant un diamètre de 67 nm fabriqué par la société dite RHONE POULENC. La fonctionnalité réactive de ce latex (dénommé ci-après "latex 1" ) est constituée par des groupes COOH et/ou COOR'' selon le pH du milieu, la teneur totale en groupes réactifs COOH et COOR'' représentant une quantité de 250 à 300 micro-équivalents par gramme de latex.

La fixation par covalence de la substance immunologique (i.e. l'antigène ou l'anticorps) est réalisée en deux étapes. L'activation préalable des fonctions carboxyle et/ou carboxylate du latex par un carbodiimide est suivie par le couplage du ligand. A 1 ml de latex à 10 % p/v on ajoute 5 mg de carbodiimide. Après incubation à 50° C ou à température ambiante (de 10 minutes à 2 heures) les billes de latex sont lavées plusieurs fois puis mises en présence de 1 à 30 mg d'antigène ou d'anticorps dilués (de préférence 5 à 10 mg) pendant 10 à 30 minutes dans un tampon biologique aqueux (tampon glycine à pH 8,35), la force ionique du milieu de contact étant de 0,01-0,5. Une substance aminée est finalement ajoutée pour saturer les sites libres sous un volume permettant une utilisation directe du réactif (0,75% p/v final).

### EXEMPLE 2

### Dosage des PDF sur sérum

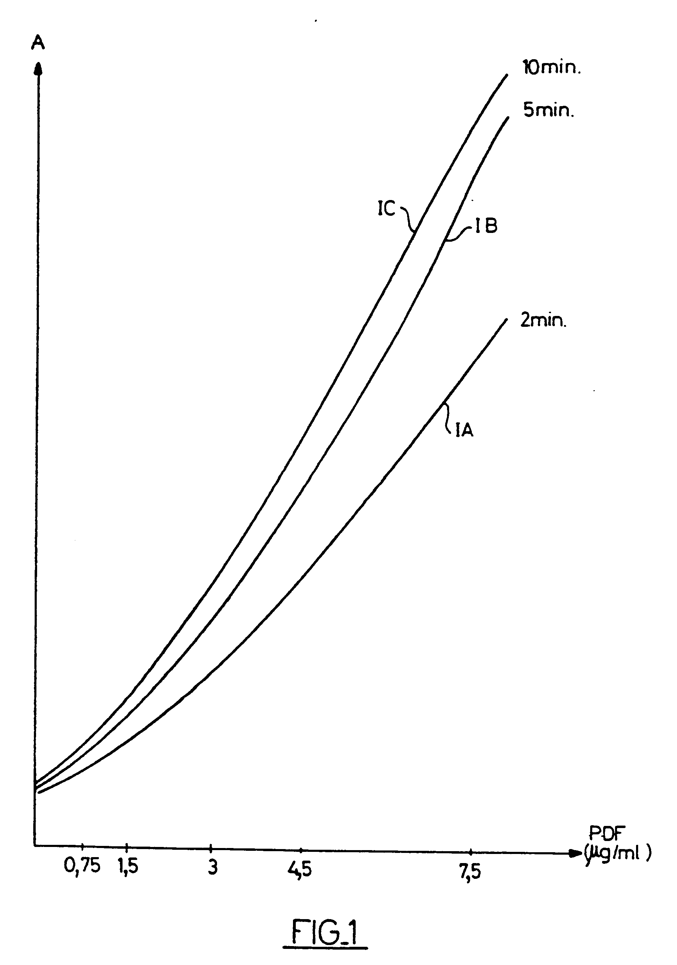

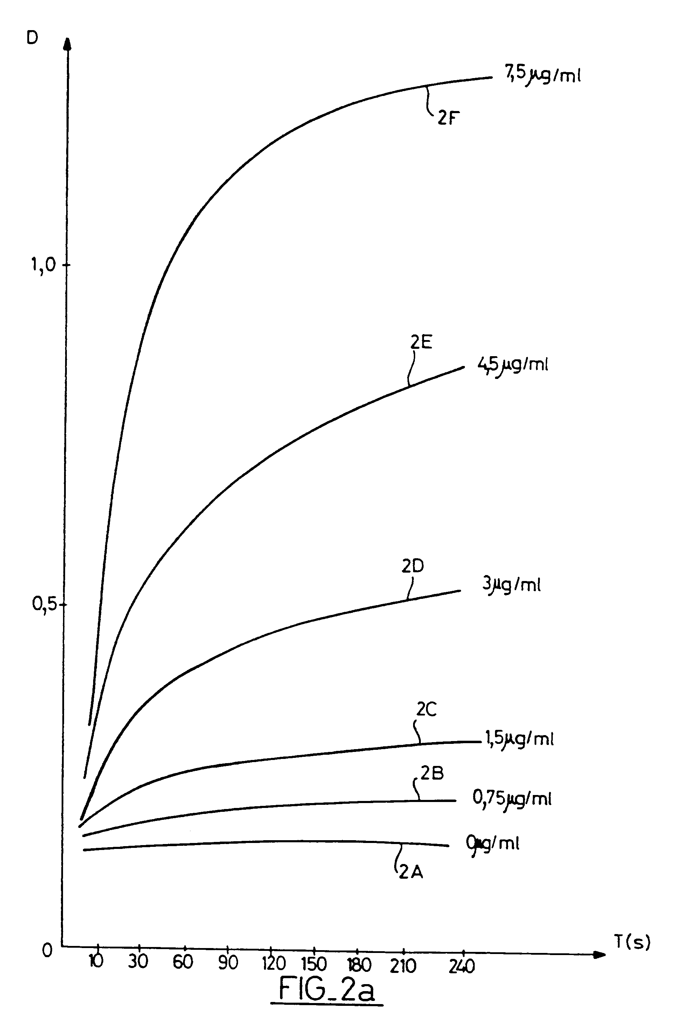

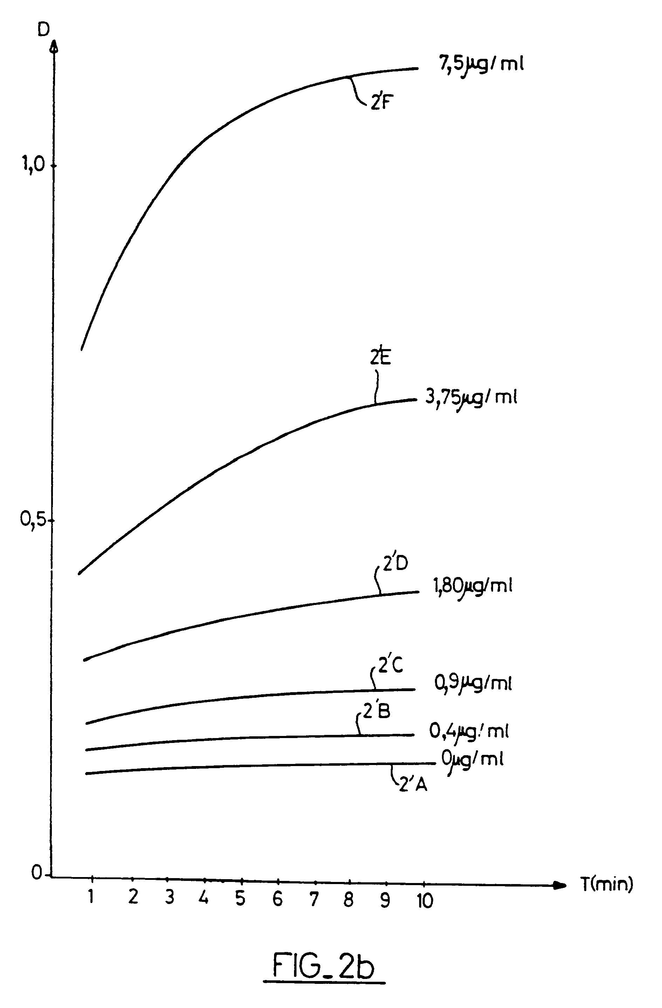

Dans la cuve de mesure sont introduits successivement 15 »l de standard ou d'échantillon à doser et 500 »l (en spectrophotométrie) ou 200 »l (sur COBAS Bio^{R}) de billes de latex revêtues d' un anticorps et diluées en tampon de réaction (concentration finale 0,075%) [préparés selon l'exemple 1 à partir d'un anticorps anti-PDF (généré selon une méthode connue en soi à partir d'un ou plusieurs PDF -Produit de dégradation du fibrinogène)]. La lecture est effectuée à 600 nm au bout de 60 à 600 s de réaction en lecture manuelle sur spectrophotomètre de 30 à 180 s an lecture automatique sur "Cobas Bio" ou par toute autre mesure cinétique. La rapidité de la réaction permet également une lecture en cinétique entre 60 s et 600 s manuellement (figure 1) ou entre 30 s et 600 s sur analyseur COBAS Bio^{R} (figures 2a et 2b). L'effet de zone est observé pour des concentrations supérieures à 500 g/ml dans l'échantillon. Une prédilution supplémentaire permet d'éliminer tout risque d'erreur par excès d'antigène. Le seuil de détection dans le milieu réactionnel se situe à 100 ng/ml.

La figure 1 donne les courbes d'étalonnage 1A, 1B et 1C pour des durées de réaction de 2,5 et respectivement de 10 minutes dans le système absorbance A (en ordonnées) / concentration finale en PDF (exprimée en »g/ml an abscisses), sous une exposition à un rayonnement de 600 nm.

La figure 2a donne les courbes des cinétiques obtenues lors du dosage des PDF dans le système densité optique D (en ordonnées) / durée de réaction T (exprimée en secondes, en abscisses) pour des concentrations finales de PDF de 0 (courbe 2A); 0,75 (courbe 2B); 1,5 (courbe 2C); 3 (courbe 2D); 4,5 (courbe 2E) et 7,5 »g/ml (courbe 2F), sous une exposition à un rayonnement de 600 nm, au moyen d'un analyseur COBAS Bio^{R}.

De même la figure 2b donne les courbes des cinétiques obtenues lors du dosage des PDF dans le système densité optique D (an ordonnées / durée de reaction T (exprimées en minutes en abscisses) pour des concentrations finales en PDF de 0 (courbe 2'A); 0,4 (courbe 2'B); 0,9 (courbe 2'C); 1,8 (courbe 2'D); 3,75 (courbe 2'E) et 7,5 »g/ml (courbe 2'F) sous une exposition à un rayonnement de 600 nm, au moyen d'un analyseur COBAS Bio^{R}.

### EXEMPLE 3

### Dosage direct de l'Antithrombine III (AT III) :

50 »l de standard ou d'échantillon prédilué 80 fois sont mélangés aux latex-anticorps [préparés selon l'exemple 1 à partir d'anti-AT III ] dilués sous un volume de 500 »l dans le tampon de réaction (0,075 % p/v final).

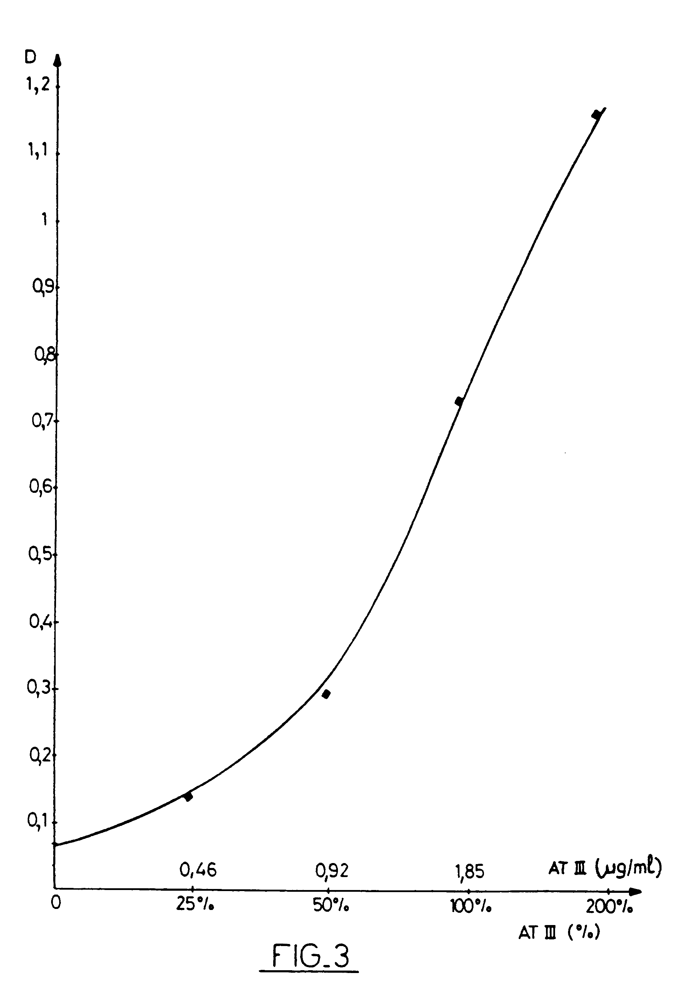

La lecture est effectuée à 600 nm après 2 à 10 minutes de réaction. La gamme d'étalonnage est comprise entre 0 et 200 % d'AT III plasmatique, ce qui correspond à des valeurs comprises entre 0 et 3,7 »g/ml dans le milieu réactionnel (figure 3).

La figure 3 donne la cours d'étalonnage à 600 nm de l'AT III plasmatique (en abscisses concentrations finales en AT III en »g/ml et en AT III plasmatique correspondante exprimée en %; avec en ordonnées la densité optique D) après 5 minutes d'incubation.

### EXEMPLE 4

### Dosage indirect des PDF

On introduit préalablement dans la cuve de mesure 200 »l d'anticorps, dans le cas d'espèce un anti-PDF, et 600 »l de PDF libres prédilués. On déclenche ensuite la réaction en ajoutant les billes de latex sensibilisées par des PDF (à 0,75%) [préparées selon l'exemple 1 à partir de PDF fixés sur lesdites billes] afin d'obtenir un volume final de 1 ml.

Les concentrations en PDF à doser, se traduisant par une inhibition de la réaction, sont exprimées en fonction de la variation de la densité optique ou de l'absorbance mesurée à 600 nm sur spectrophotomètre.

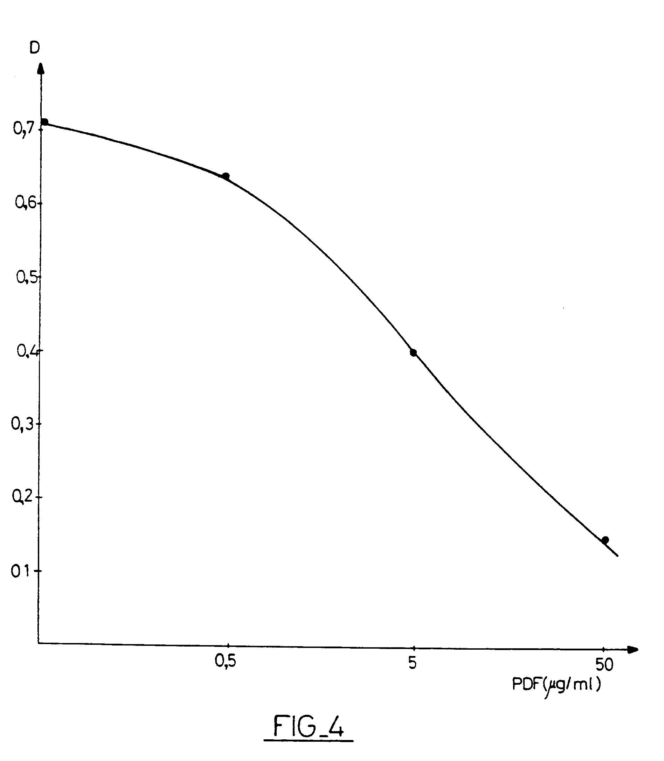

La figure 4 ci-après donne la courbe d'inhibition de l'agglutination du réactif latex/PDF dans le système densité optique D (en ordonnées), concentration des PDF (exprimée en »g/ml en abscisse) à 600 nm.

### EXEMPLE 5

### Préparation du réactif immunologique

On fait appel au latex 1 de l'exemple 1, sous la forme de billes ayant un diamètre de 67 nm, la fonctionnalité réactive de ce latex représentant, comme indiqué à l'exemple 1, une quantité de 250 à 300 micro-équivalents de groupes carboxylés par gramme de latex.

La fixation par adsorption de la substance immunologique (i.e. l'antigène ou l'anticorps) est effectuée à la surface du matériau polymère des particules. On met en contact dans un tampon glycine à pH 8,35 100 mg (1 ml à la concentration de 10 % p/v) de particules submicroniques du latex 1 avec 1 à 50 mg (de préférence 2 à 20 mg) d'anticorps (ou d'antigène) de haute pureté, le milieu biologique d'adsorption ayant une activité ionique de 0,01-0,5. On incube à 56°C pendant 1-24 h.

On rince les particules submicroniques sur lesquelles a été fixée la substance immunologique puis après mise en suspension dans le même tampon glycine à pH 8,35, on ajoute 20 à 40 mg de matériau polyhydroxylé, de préférence ici le diéthylèneglycol, l'éthylèneglycol, le glycérol ou le PEG; on incube ensuite à 56°C pendant 1-24 h.

On dilue ensuite la suspension résultante à la dilution d'utilisation (ici 0,075 % p/v) au moyen du tampon glycine de stabilisation contenant ledit matériau polyhydroxylé (ici le diéthylèneglycol, l'éthylèneglycol, le glycérol ou le PEG) et de l'albumine.

### EXEMPLE 6

### Dosage des PDF sur sérum

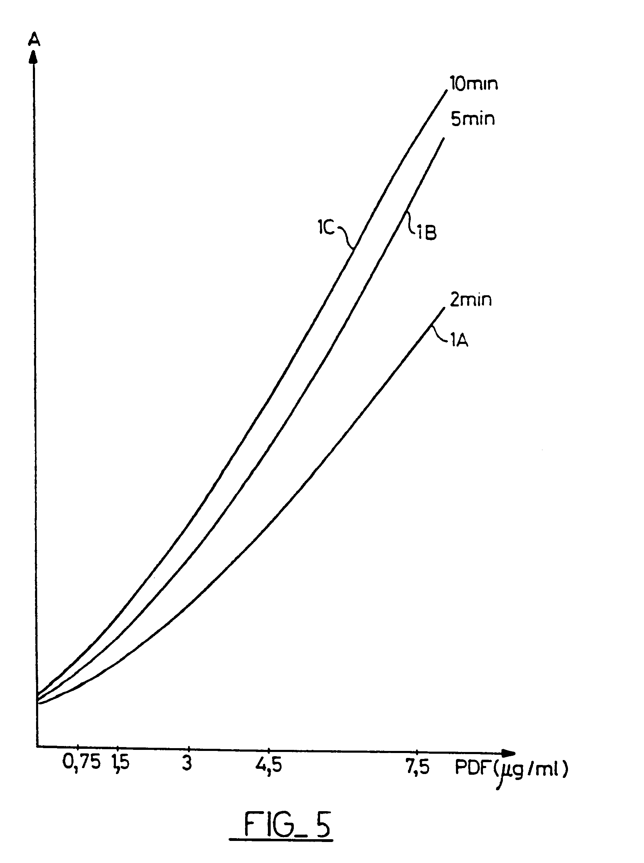

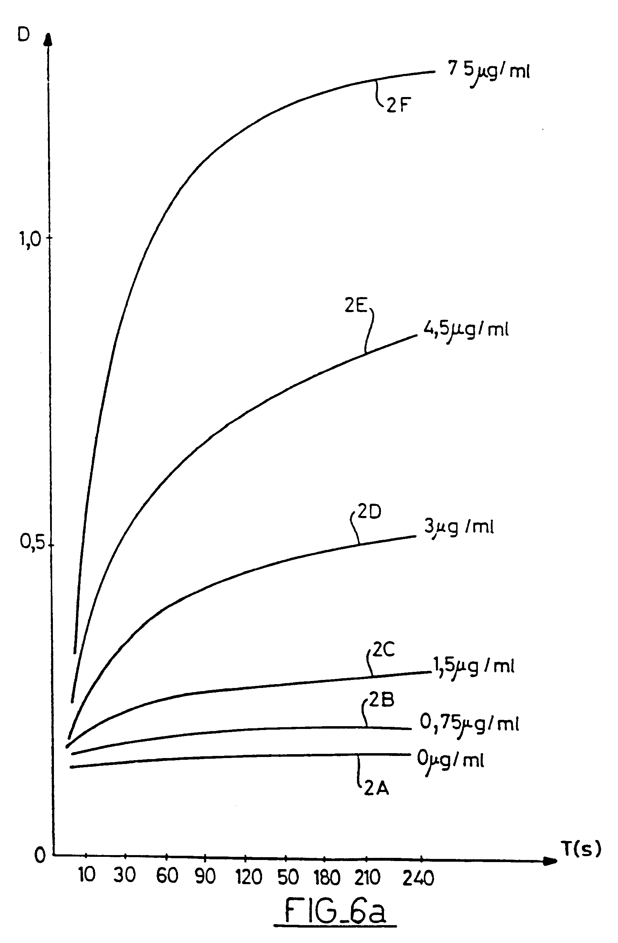

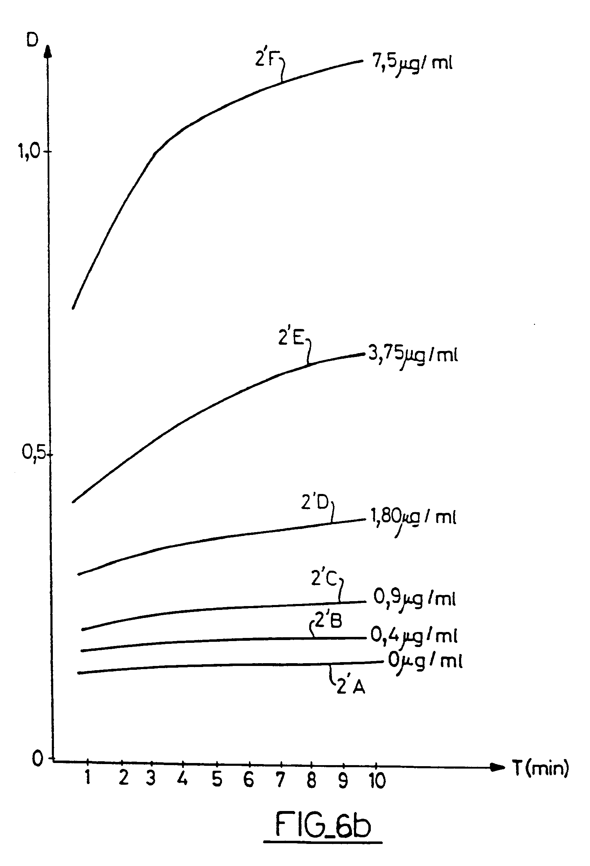

Dans la cuve de mesure sont introduits successivement 15 »l de standard ou d'échantillon à doser et 500 »l (en spectrophotométrie) ou 200 »l (sur COBAS Bio^{R}) de billes de latex revêtues d'un anticorps et diluées en tampon de réaction (concentration finale 0,075 %) [préparées selon l'exemple 5 à partir d'un anticorps anti-PDF, généré selon une méthode connue en soi à partir d'un ou plusieurs PDF (produit de dégradation du fibrinogène)]. La lecture est effectuée à 600 nm au bout de 60 à 600 s de réaction en lecture manuelle sur spectrophotomètre ou de 30 à 180 s en lecture automatique sur COBAS Bio^{R} ou par toute autre mesure cinétique. La rapidité de la réaction permet également une lecture en cinétique entre 60 s et 600 s manuellement (figure 5), ou entre 30 s et 600 s sur analyseur COBAS Bio^{R} (figures 6a et 6b). L'effet de zone est observé tour des concentrations supérieures à 500 »g/ml dans l'échantillon. Une prédilution supplémentaire permet d'éliminer tout risque d'erreur par excès d'antigène. Le seuil de détection dans le milieu réactionnel se situe à 100 ng/ml.

La figure 5 donne les courbes d'étalonnage 1A, 1B et 1C pour des durées de réaction de 2,5 et respectivement de 10 minutes dans le système absorbance A (en ordonnées) / concentration finale en PDF exprimée en »g/ml (en abscisses), sous une exposition à un rayonnement de 600 nm.

La figure 6a donne les courbes des cinétiques obtenues lors du dosage des PDF dans le système densité optique D (en ordonnées) / durée de réaction T exprimée en secondes (en abscisses) pour des concentrations finales de PDF de 0 (courbe 2A); 0,75 (courbe 2B); 1,5 (courbe 2C); 3 (courbe 2D); 4,5 (courbe 2E) et 7,5 »g/ml (courbe 2F), sous une exposition à un rayonnement de 600 nm, au moyen d'un analyseur COBAS Bio^{R}.

De même la figure 6b donne les courbes des cinétiques obtenues lors du dosage des PDF dans le système densité optique D (en ordonnées) / durée de réaction T exprimée en minutes (en abscisses) pour des concentrations finales en PDF de 0 (courbe 2'A); 0,4 (courbe 2'B); 0,9 (courbe 2'C); 1,8 (courbe 2'D); 3,75 (courbe 2'E) et 7,5 »g/ml (courbe 2'F) sous une exposition à un rayonnement de 600 nm, au moyen d'un analyseur COBAS Bio^{R}.

### EXEMPLE 7

### Dosage direct de l'Antithrombine III (AT III) :

50 »l de standard ou d'échantillon prédilué 80 fois sont mélangés aux latex-anticorps [préparés selon l'exemple 5 à partir d'anti-AT III ] dilués sous un volume de 500 »l dans le tampon de réaction (0,075 % p/v final).

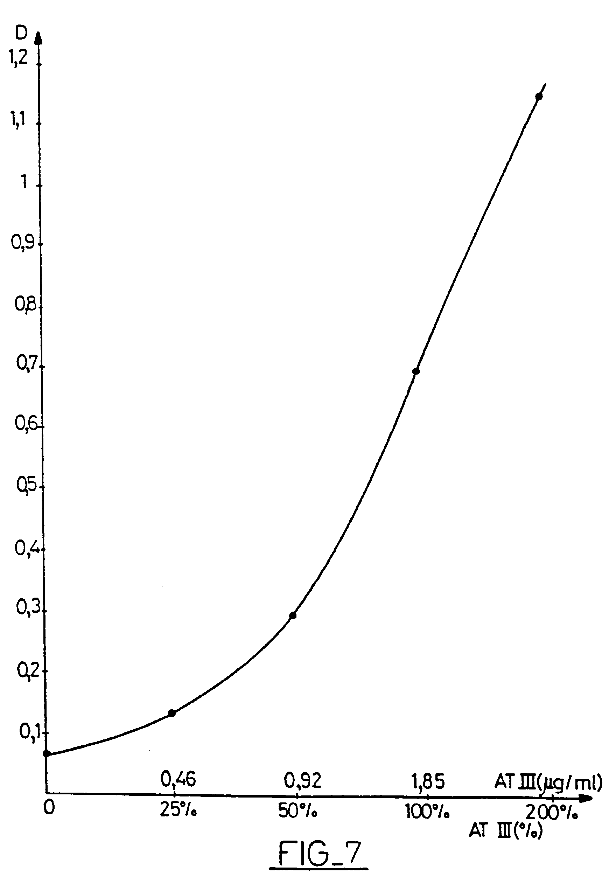

La lecture est effectuée à 600 nm après 2 à 10 minutes de réaction. La gamme d'étalonnage est comprise entre 0 et 200 % d'AT III plasmatique, ce qui correspond à des valeurs comprises entre 0 et 3,7 »g/ml dans le milieu réactionnel (figure 7).

La figure 7 donne la courbe d'étalonnage à 600 nm de l'AT III plasmatique (en abscisses : concentrations finales en AT III en »g/ml et en AT III plasmatique correspondante exprimée en %; avec en ordonnées : la densité optique D) après 5 minutes d'incubation.

### EXEMPLE 8

### Dosage indirect des PDF

On introduit préalablement dans la cuve de mesure 200 »l d'anticorps, dans le cas d'espèce un anti-PDF, et 600 »l de PDF libres prédilués. On déclenche ensuite la réaction en ajoutant les billes de latex sensibilisées par des PDF (à 0,75 %) [préparées selon l'exemple 5 par adsorption de PDF sur lesdites billes] afin d'obtenir un volume final de 1 ml.

Les concentrations en PDF à doser, se traduisant par une inhibition de la réaction, sont exprimées en fonction de la variation de la densité optique ou de l'absorbance mesurée à 600 nm sur spectrophotomètre.

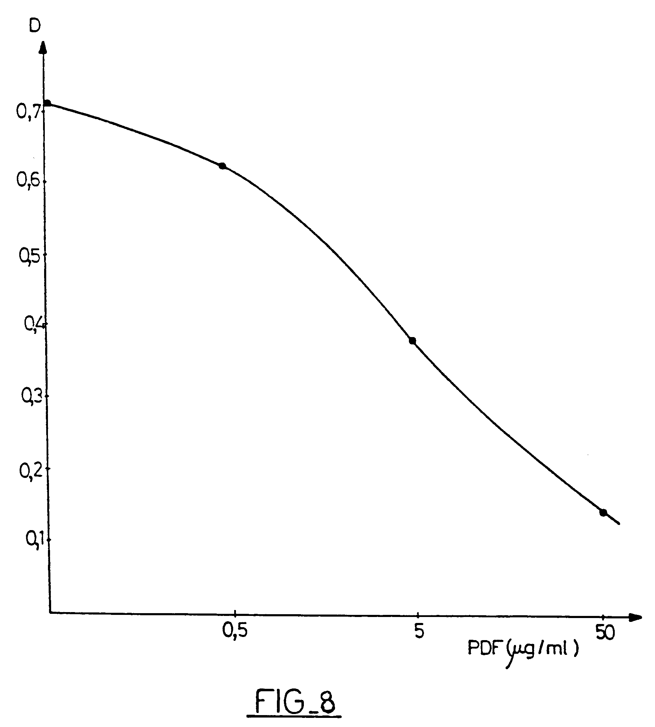

La figure 8 ci-après donne la courbe d'inhibition de l'agglutination du réactif latex/PDF dans le système densité optique D (en ordonnées), concentration des PDF exprimée en »g/ml (en abscisse) à 600 nm.

Conformément à l'invention on préconise des nécessaires ou trousses de dosages contenant notamment en suspension dans un liquide aqueux (i) des microsphères de latex destinées à être liées par adsorption avec la substance immunologique de l'utilisateur, ou (ii) des réactifs immunologiques dans lesquels la substance immunologique est adsorbée sur chacune des microsphères pour constituer, notamment un réactif immunologique utile en particulier dans le domaine de l'hémostase, tel que latex/PDF, latex/anti-PDF, latex/anti-AT III ou latex/AT III.

### ESSAIS COMPARATIFS

On a entrepris deux séries d'essais comparatifs avec des réactifs immunologiques élaborés à partir des latex carboxylés suivants (ayant une teneur en groupes COOH ou COOR' de 250-350 micro-équivalents), sous forme de billes ayant un diamètre de 55 à 70 nm (les teneurs respectives des comonomères sont exprimées en pourcentage molaire) :
- Latex A :: polypropylène
- Latex B :: styrène-méthacrylate de butyle (52/48)
- Latex C :: acrylate de méthyle-méthacrylate de butyle (55/45)
- Latex D :: styrène-butadiène (55/45)
- Latex E :: styrène-butadiène-méthacrylate de butyle (50/25/25)
sur lesquels on a fixé des anticorps anti-PDF par covalence (série A) ou par adsorption (série B) afin de les comparer avec les réactifs immunologiques obtenus à partir desdits anticorps anti-PDF et du latex 1 par covalence (exemple 1) et respectivement par adsorption (exemple 5).

On a apprécié la stabilité lors du stockage à 4°C pendant au plus 180 jours à la concentration de 1 % p/v. Les résultats obtenus figurent dans les tableaux A et B suivants correspondant respectivement aux séries (A) et (B).

**TABLEAU A**

| **STABILITE DE REACTIFS IMMUNOLOGIQUES DANS LESQUELS LE MATERIEL IMMUNOLOGIQUE EST FIXE PAR COVALENCE** | |
|---|---|
| Latex | Auto-agglutination |
| Latex 1 | pas d'agglutination à J = 180 |
| Latex A | auto-agglutination à J = 110 |
| Latex B | auto-agglutination à J = 150 |
| Latex C | auto-agglutination à J = 142 |
| Latex D | auto-agglutination à J = 108 |
| Latex E | auto-agglutination à J = 137 |

**TABLEAU B**

| **STABILITE DE REACTIFS IMMUNOLOGIQUES DANS LESQUELS LE MATERIEL IMMUNOLOGIQUE EST FIXE PAR ADSORPTION** | |
|---|---|
| Latex | Auto-agglutination |
| Latex 1 | pas d'agglutination à J = 180 |
| Latex A | auto-agglutination à J = 115 |
| Latex B | auto-agglutination à J = 145 |
| Latex C | auto-agglutination à J = 138 |
| Latex D | auto-agglutination à J = 116 |
| Latex E | auto-agglutination à J = 128 |

Les résultats des tableaux A et B mettent en évidence l'intérêt du choix du latex dans la stabilité du réactif immunologiques selon l'invention, le latex 1 ne donnant lieu à aucune auto-agglutination pendant une durée d'au moins 180 jours.

## Revendications

1. Procédé de fixation par covalence ou adsorption d'un matériel immunologique choisi parmi les antigènes et les anticorps sur des particules submicroniques de latex, ledit procédé, qui comporte la stabilisation du latex, étant caractérisé en ce qu'il comprend
(A) la mise en contact dans un milieu liquide approprié ayant une force ionique comprise dans la gamme de 0,01 à 0,5 à un pH de 4 à 10, à une température de 0 à 60°C
(1) de particules de latex acrylique à base de polyméthacrylate de butyle, ayant un diamètre moyen inférieur ou égal à 100 nm, ledit latex étant un copolymère constitué de motifs styrène et de motifs méthacrylate de butyle dans un rapport molaire motifs styrène/motifs méthacrylate de butyle de 40/60 à 45/55, renfermant une quantité molaire de motifs méthacrylate de butyle supérieure à 50 %, ayant une densité de 0,9 à 1,4 g/cm³ et comportant de 100 à 450 micro-équivalents de groupes COOH par gramme de latex, avec
(2) le matériel immunologique
pour obtenir des particules de latex sensibilisées par un matériel immunologique et telles que ledit matériel immunologique soit lié à chaque particule de latex, et
(B) la stabilisation dudit réactif immunologique ainsi obtenu au moyen d'un matériau stabilisant choisi parmi l'ensemble constitué par les substances hydroxylées contenant un ou mieux plusieurs groupes OH par molécule, les substances protéiniques, les substances peptidiques, les aminoacides, les substances polyosiques comportant un ou plusieurs groupes acide carboxylique et/ou carboxyate, la polyvinylpyrrolidone et leurs mélanges.

2. Procédé suivant la revendication 1, caractérisé en ce que ledit latex acrylique est un copolymère constitué de motifs styrène et de motifs méthacrylate de butyle dans un rapport molaire motifs styrène/motifs méthacrylate de butyle de 42/58 à 43/57 et en ce que ledit latex acrylique a une densité de 1,0 à 1,1 g/cm³ et contient de 250 à 300 micro-équivalents de groupes COOH par gramme de latex.

3. Procédé suivant la revendication 1, caractérisé en ce que la mise en contact du point (A) est réalisée dans un milieu liquide comprenant ledit matériau stabilisant du point (B).

4. Procédé suivant la revendication 1, caractérisé en ce que la stabilisation du point (B) est réalisée après la mise en contact du point (A).

5. Procédé suivant la revendication 1, caractérisé en ce que lesdites particules de latex ont un diamètre moyen compris entre 10 et 100 nm.

6. Procédé suivant la revendication 1, pour la fixation par covalence dudit matériel immunologique sur lesdites particules de latex, caractérisé en ce qu'il comprend la mise en contact pendant au moins 10 minutes dans un milieu aqueux tamponné à un pH de 4-10 et ayant une force ionique de 0,01 à 0,5 dudit matériel immunologique avec lesdites particules de latex dans lesquelles les sites actifs COOH ont été préalablement sensibilisés au moyen d'un composé carbodiimide, puis l'addition d'un matériau stabilisant en excès pour bloquer les sites actifs encore libres sur lesdites particules desdites particules de latex.

7. Procédé suivant la revendication 6, caractérisé en ce qu'il comprend, avant la mise en contact, la réaction des sites actifs desdites particules de latex avec un composé bifonctionnel extendeur de chaîne dans un milieu aqueux tamponné à pH 4-10 et ayant une force ionique de 0,01 à 0,5 à une température de 40-60°C pendant au moins 10 minutes.

8. Procédé suivant la revendication 6, caractérisé en ce qu'il comprend la mise en contact de particules de latex comportant en surface une couche moléculaire de liaison en un matériau choisi parmi l'albumine et la polylysine.

9. Procédé suivant la revendication 1 pour la fixation par adsorption dudit matériel immunologique sur lesdites particules de latex, caractérisé en ce qu'il comprend la mise en contact, pendant au moins 1 h, dans un milieu aqueux tamponné à un pH de 4-10 et ayant une force ionique de 0,01 à 0,5, dudit matériel immunologique avec lesdites particules de latex, puis l'addition d'un matériau stabilisant en excès pour bloquer les sites actifs encore libres sur lesdites particules desdites particules de latex.

10. Procédé suivant l'une quelconque des revendications 1, 6 et 9, caractérisé en ce que la mise en contact dudit matériel immunologique avec lesdites particules de latex est réalisée dans un tampon glycine à pH 8,35.

11. Procédé suivant la revendication 1, caractérisé en ce que l'on met en contact selon le point (A) 100 parties en poids de particules de latex avec 1 à 50 et de préférence 2 à 20 parties en poids de matériel immunologique, et en ce que l'on utilise 10 à 100 parties en poids de matériau stabilisant pour 100 parties en poids de particules de latex utilisées lors de ladite mise en contact.

12. Procédé suivant la revendication 6, caractérisé en ce que les sites actifs COOH et respectivement COOR' des particules de latex sont préalablement sensibilisés par un composé carbodiimide et respectivement un composé glutaraldéhyde.

13. Réactif immunologique constitué par des particules submicroniques de latex sur lesquelles a été fixé par covalence ou adsorption un matériel immunologique, caractérisé en ce qu'il est obtenu selon le procédé de l'une quelconque des revendications 1, 6, 9 et 11, et en ce que lesdites particules sont telles que
(i) revêtues de ladite substance immunologique et dudit matériau stabilisant mises en suspension dans un milieu liquide aqueux, elles sont invisibles quand elles sont exposées à un rayonnement ayant une longueur d'onde (λ) supérieure au diamètre moyen (d) desdites particules, et
(ii) revêtues de ladite substance immunologique et dudit matériau stabilisant mises en suspension dans ledit milieu liquide aqueux, elles deviennent "visibles" par agglutination quand elles sont exposées audit rayonnement après mise en contact avec une substance choisie parmi les anticorps et respectivement les antigènes et qui correspond à ladite substance immunologique fixée sur lesdites particules,
le rapport λ/d étant supérieur ou égal à 5/1.

14. Procédé de dosage mettant en oeuvre une réaction du type antigène/anticorps, caractérisé en ce qu'il consiste
(1) à mettre en contact, dans un milieu liquide aqueux ayant un pH situé dans la gamme de 4 à 10 et une force ionique située dans la gamme de 0,01 à 0,5, (i) des particules de latex, ledit latex étant un copolymère constitué de motifs styrène et de motifs méthacrylate de butyle dans un rapport molaire motifs styrène/motifs méthacrylate de butyle de 40/60 à 45/55, renfermant une quantité molaire de motifs méthacrylate de butyle supérieure à 50 %, ayant une densité de 0,9 à 1,4 g/cm³ et comportant de 100 à 450 micro-équivalents de groupes COOH par gramme de latex, lesdites particules ayant un diamètre moyen (d) inférieur ou égal à 100 nm et étant revêtues d'une substance immunologique choisie parmi les antigènes et les anticorps et liée par covalence ou adsorption à chacune desdites particules, les sites actifs de chacune desdites particules non liés à ladite substance immunologique étant essentiellement bloqués par un matériau stabilisant choisi parmi l'ensemble constitué par les substances hydroxylées contenant un ou mieux plusieurs groupes OH par molécule, les substances protéiniques, les substances peptidiques, les aminoacides, les substances polyosiques comportant un ou plusieurs groupes acide carboxylique et/ou carboxylate, la polyvinylpyrrolidone, leurs analogues et leurs mélanges, avec (ii) une substance complémentaire ou conjuguée de ladite substance immunologique, et
(2) à apprécier l'évolution de l'agglutination par exposition à un rayonnement ayant une longueur d'onde (λ) située dans la gamme de 300 à 1000 nm (notamment 400-700 nm), telle que le rapport λ/d soit supérieur ou égal à 5.

## Claims

1. A method of binding an immunological material selected from antigens and antibodies, to submicron latex particles by covalency or adsorption, said method, which comprises the stabilization of the latex, being characterized in that it comprises,
(A) bringing into contact, in an appropriate liquid medium having an ionic strength in the range from 0.01 to 0.5 at a pH of 4 to 10, at a temperature of 0 to 60°C,
(1) acrylic latex particles based on polybutyl methacrylate, whose mean diameter is less than or equal to 100 nm, said latex being constituted of styrene units and butyl methacrylate units, having a molar ratio styrene units/butyl methacrylate units of 40/60 to 45/55, containing a molar quantity of butyl methacrylate units greater than 50 %, having a density of 0.9 to 1.4 g/cm³ and containing from 100 to 450 microequivalents of COOH groups per gram of latex, with
(2) the immunological material
to give latex particles sensitized by an immunological material and such that said immunological material is bound to each latex particle, and
(B) stabilizing said immunological reagent thus obtained by means of a stabilizing material selected from the group consisting of hydroxylated substances containing one or several OH groups per molecule, protein-type substances, peptide-type substances, amino acids, polyose-type substances containing one or more carboxylic acid and/or carboxylate groups, polyvinylpyrrolidone and mixtures thereof.

2. A method according to claim 1, wherein said acrylic latex is a copolymer consisting of styrene units and butyl methacrylate units in a molar ratio styrene units/butyl methacrylate units of 42/58 to 43/57, and wherein said acrylic latex has a density of 1.0 to 1.1 g/cm³ and contains from 250 to 300 microequivalents of COOH groups per gram of latex.

3. A method according to claim 1, wherein the contacting process of section (A) is carried out in a liquid medium comprising said stabilizing material of section (B).

4. A method according to claim 1, wherein the stabilization of section (B) is carried out after the contacting process of section (A).

5. A method according to claim 1 wherein said latex particles have a mean diameter of between 10 and 100nm.

6. A method according to claim 1 for covalent binding of said immunological material to said latex particles, which comprises bringing said immunological material into contact, for at least 10 minutes, in an aqueous medium buffered to a pH of 4-10 and having an ionic strength of 0.01 to 0.5, whith said latex particles in which the active COOH sites have been sensitized beforehand by means of a carbodiimide compound, and then adding an excess of a stabilizing material to block the active sites which are still free on said latex particles.

7. A method according to claim 6, which comprises, prior to the contacting process, reacting the active sites of said latex particles with a difunctional chain-extending compound, in an aqueous medium buffered to pH 4-10 and having an ionic strength of 0.01 to 0.5, at a temperature of 40-60°C, for at least 10 minutes.

8. A method according to claim 6, which comprises carrying out the contacting process with latex particles containing, on the surface, a molecular binding layer of a material selected from albumin and polylysine.

9. A method according to claim 1 for binding of said immunological material to said latex particles by adsorption, which comprises bringing said immunological material into contact with said latex particles for at least 1 h, in an aqueous medium buffered to a pH of 4-10 and having an ionic strength of 0.01 to 0.5, and then adding an excess of a stabilizing material to block the active sites which are still free on said latex particles.

10. A method according to any one of claims 1, 6 and 9, wherein said immunological material is brought into contact with said latex particles in a glycine buffer at pH 8.35.

11. A method according to claim 1, wherein 100 parts by weight of latex particles are brought into contact, in accordance with section (A), with 1 to 50 and preferably, 2 to 20 parts by weight of immunological material, and wherein 10 to 100 parts by weight of stabilizing material are used per 100 parts by weight of latex particles used in said contacting process.

12. A method according to claim 6, wherein the active COOH and respectively COOR sites of the latex particles are sensitized beforehand with a carbodiimide compound and respectively a glutaraldehyde compound.

13. An immunological reagent consisting of submicron latex particles to which an immunological material has been bound by covalency or adsorption, said reagent being characterized in that it is obtained by the method of any one of claims 1, 6, 9 and 11, and in that said particles are such that
(i) coated with said immunological substance and said stabilizing material and suspended in an aqueous liquid medium, there are invisible when exposed to radiation with a wavelength (λ) greater than the mean diameter (d) of said particles, and
(ii) coated with said immunological substance and said stabilizing material and suspended in said aqueous liquid medium, they become "visible" due to agglutination when exposed to said radiation after having been brought into contact with a substance selected from antibodies and respectively antigens and corresponding to said immunological substance bound to said particles, the ratio λ/d being greater than or equal to 5/1.

14. An assay method involving a reaction of the antigen/antibody type, which consists in
(1) bringing into contact, in an aqueous liquid medium having a pH in the range from 4 to 10 and an ionic strength in the range from 0.01 to 0.5 (i) latex particles, said latex being constituted of styrene units and butyl methacrylate units, having a molar ratio styrene units/butyl methacrylate units of 40/60 to 45/55, containing a molar quantity of butyl methacrylate units greater than 50 %, having a density of 0.9 to 1.4 g/cm³ and containing from 100 to 450 microequivalents of COOH groups per gram of latex, and said particles having a mean diameter (d) lower than or equal to 100 nm and being coated with an immunological substance selected from antigens and antibodies and bound to each of said particles by covalency or adsorption, the active sites of each of said particles which are not bound to said immunological substance being essentially blocked by a stabilizing material selected from the group consisting of hydroxylated substances containing one or, preferably, several OH groups per molecule, protein-type substances, peptide-type substances, amino acids, polyose-type substances containing one or more carboxylic acid and/or carboxylate groups, polyvinylpyrrolidone, analogs thereof and mixtures thereof, whith (ii) a complementary or conjugate substance for said immunological substance, and
(2) assessing the change in agglutination by exposure to radiation with a wavelength (λ) in the range from 300 to 1000 nm (especially 400-700 nm) and such that the ratio λ/d is greater than or equal to 5.

## Patentansprüche

1. Verfahren zum Fixieren von immunologischem, unter Antigenen und Antikörpern auf submikronischen Latexteilchen ausgewähltem Material mittels Kovalenz oder Adsorption, wobei das Verfahren, welches die Stabilisierung des Latex beinhaltet, dadurch gekennzeichnet ist, daß es folgende Schritte umfaßt
(A) In einem geeigneten, flüssigen Milieu mit einer ionischen Kraft im Bereich von 0,01 bis 0,5 bei einem pH von 4 bis 10, und einer Temperatur von 0 bis 60° C, Verbindung
(1) von Latexacrylteilchen auf Butylpolymethacrylatbasis mit einem mittleren Durchmesser, der kleiner oder gleich 100 nm ist, wobei das Latex ein aus Styroleinheiten und aus Butylmethacrylateinheiten bestehender Copolymer ist, mit einem molaren Verhältnis Styroleinheit/Butylmethacrylateinheit von 40/60 bis 45/55, einschließlich einer molaren Menge von Butylmethacrylateinheiten mit über 50 %, bei einer Dichte von 0,9 bis 1,4 g/cm2, die aus 100 bis 450 Mikroäquivalenten der COOH-Gruppen pro Gramm Latex zusammengesetzt sind, mit
(2) dem immunologischen Material
um durch ein immunologisches Material sensibilisierte Latexpartikel zu gewinnen und wobei das immunologische Material mit jedem Latexpartikel verbunden ist und
(B) Stabilisierung des so gewonnenen, immunologischen Reagens, mittels eines stabilisierenden Materials, das aus dem gesamten Komplex, bestehend aus den hydroxylierten Substanzen mit einer oder mehreren OH-Gruppen pro Molekül, aus den Eiweißstoffen, den Peptiden, den Aminosäuren, den polyosischen Substanzen, einschließlich einer oder mehrerer Carboxylsäuren und/oder Carboxyatgruppen, dem Polyvinylpyrrolidon und seinen Gemischen besteht, ausgewählt wird.

2. Verfahren gemäß Anspruch 1, dadurch gekennzeichnet, daß das Latexacryl ein Copolymer ist, der aus Styroleinheiten und Butylmethacrylateinheiten mit einem molaren Verhältnis Styroleinheit/Butylmethacrylateinheit von 42/58 bis 43/57 und wobei das Latexacryl eine Dichte von 1,0 bis 1,1 g/cm2 hat und zwischen 250 bis 300 Mikroäquivalente der COOH Gruppen pro Gramm Latex aufweißt.

3. Verfahren gemäß Anspruch 1, dadurch gekennzeichnet, daß die Verbindung aus Punkt (A) in einem flüssigen Milieu geschieht, welches das stabilisierende Material aus Punkt (B) enthält.

4. Verfahren gemäß Anspruch 1, dadurch gekennzeichnet, daß die Stabilisierung aus Punkt (B) nach der Verbindung aus Punkt (A) geschieht.

5. Verfahren gemäß Anspruch 1, dadurch gekennzeichnet, daß die Latexpartikel einen mittleren Durchmesser von 10 bis 100 nm aufweisen.

6. Verfahren gemäß Anspruch 1, zum Fixieren des immunologischen Materials auf den Latexteilchen mittels Kovalenz, dadurch gekennzeichnet, daß es die Verbindung von mindestens 10 Minuten in einem wässrigen Pufferlösung mit einem pH von 4-10 und einer ionischen Kraft von 0,01 bis 0,5 des immunologischen Materials mit den Latexteilchen beinhaltet, in denen die aktiven COOH Stellen vorab mittels einer Carbodiimidverbindung sensibilisiert wurden, und anschließend Hinzufügen eines stabilisierenden Materials in überschüssiger Menge zur Blockierung der auf den Partikeln der Latexpartikel noch freien, aktiven Stellen.

7. Verfahren gemäß Anspruch 6, dadurch gekennzeichnet, daß es vor der Verbindung, die Reaktion der aktiven Stellen der Latexpartikel mit einer bifunktionellen, kettenausdehnenden Verbindung in einer wässrigen Pufferlösung bei einem pH von 4-10 und mit einer ionischen Kraft von 0,01 bis 0,5 bei einer Temperatur von 40-60° C, mindestens 10 Minuten lang, beinhaltet.

8. Verfahren gemäß Anspruch 6, dadurch gekennzeichnet, daß es die Verbindung der Latexpartikel einschließt, die an ihrer Oberfläche eine molekulare Verbindungsschicht aufweisen, welche aus einem aus Albumin und Polylysin ausgewählten Material besteht.

9. Verfahren gemäß Anspruch 1 zum Fixieren des immunologischen Materials auf den Latexteilchen mittels Adsorption, dadurch gekennzeichnet, daß es die mindestens einstündige Verbindung des immunologischen Materials mit den Latexteilchen umfaßt, in einem wässrigen Milieu bei einem pH von 4-10 und einer ionischen Kraft von 0,01 bis 0,5, und anschließend das Hinzufügen eines stabilisierenden Materials in überschüssiger Menge zur Blockierung der aktiven, noch auf den Partikeln der Latexpartikel freien Stellen.

10. Verfahren gemäß irgendeinem der Ansprüche 1, 6 und 9 dadurch gekennzeichnet, daß die Verbindung des immunologischen Materials mit den Latexteilchen in einem Glyzinpuffer mit einem pH von 8,35 geschieht.

11. Verfahren gemäß Anspruch 1, dadurch gekennzeichnet, daß man gemäß Punkt (A) 100 Gewichtsanteile von Latexteilchen mit 1 bis 50 und vorzugsweise 2 bis 20 Gewichtsanteilen von immunologischem Material miteinander in Kontakt bringt, dadurch gekennzeichnet, daß man 10 bis 100 Gewichtsanteile von stabilisierendem Material für 100 Gewichtsanteile von Latexteilchen verwendet, die während der Verbindung verwendet wurden.

12. Verfahren gemäß Anspruch 6, dadurch gekennzeichnet, daß die aktiven COOH Stellen, beziehungsweise COOR' der Latexpartikel vorab durch eine Carbodiimidverbindung beziehungsweise durch eine Glutaraldehydverbindung sensibilisiert wurden.

13. Immunologisches Reagens, bestehend aus submikronischen Latexteilchen, auf denen durch Kovalenz oder Adsorption ein immunologisches Material fixiert wurde, dadurch gekennzeichnet, daß es gemäß dem Verfahren aus einem der Ansprüche 1, 6, 9 und 11 gewonnen wurde und wobei die Partikel derart beschaffen sind, daß sie
(i) mit der immunologischen Substanz und dem stabilisierenden Material versehen sind, sie sich in einer Suspension in einem flüssigen, wässrigen Milieu befinden, sie unsichtbar sind, wenn sie mit einer Wellenlänge (A), über dem mittleren Durchmesser (d) der Partikel bestrahlt werden und
(ii) mit der immunologischen Substanz versehen sind und das stabilisierende Material sich in einer Suspension in dem flüssigen, wässrigen Milieu befindet, sie durch Agglutination "sichtbar" werden, wenn sie bestrahlt werdem, sie mit einer Substanz in Kontakt gebracht wurden, die unter den Antikörpern beziehungsweise den Antigenen ausgewählt wurde und die der auf den Partikeln befestigten Substanz entspricht, wobei das Verhältnis A/d über oder gleich 5/1 ist.

14. Verfahren zur Dosierung unter Verwendung einer Reaktion des Typs Antigen/Antikörper, dadurch gekennzeichnet, daß es
(1) in einem flüssigen, wässrigen Milieu bei einem pH im Bereich zwischen 4 und 10 und einer ionischen Stärke im Bereich von 0,01 bis 0,5 (i) Latexpartikel in Kontakt bringt, wobei das Latex ein Copolymer ist, der aus Styroleinheiten und Butylmethacrylateinheiten besteht, bei einem molaren Verhältnis Styroleinheit/Butylmethacrylateinheit von 40/60 bis 45/55, einschließlich einer molaren Menge von Butylmetharylateinheiten über 50% mit einer Dichte von 0,9 bis 1,4 g/cm2 und die 100 bis 450 Mikroäquivalente der COOH-Gruppen pro Gramm Latex aufweisen, wobei die Partikel einen mittleren Durchmesser (d) von weniger oder gleich 100 nm aufweisen und mit einer immunologischen Substanz versehen sind, die unter den Antigenen und Antikörpern ausgewählt wurde und die durch Kovalenz oder Adsorption mit jedem Partikel verbunden ist, wobei die aktiven Stellen jedes nicht mit der immunologischen Substanz verbundenen Partikels, hauptsächlich von einem stabilisierenden Material blockiert werden, das aus der Verbindung bestehend aus den hydroxylischen Substanzen ausgewählt wurde, die eine oder mehrere OH-Gruppen pro Molekül aufweisen, wobei die Eiweißstoffe, Peptide, Aminosäuren, die polyosischen Substanzen aus einer oder aus mehreren Gruppen von Carboxyl- und/oder Carboxylatsäuren, dem Polyvinylpyrrolidon, deren Analogen oder deren Gemischen, mit (ii) einer zusätzlichen oder der immunologischen Substanz zugeordneten Substanz bestehen, und
(2) zur Beurteilung der Entwicklung der Agglutination durch Bestrahlung bei einer Wellenlänge (A) im Bereich von 300 bis 1000 nm (vorwiegend 400-700 nm), sodaß das Verhältnis A/d mehr oder gleich 5 beträgt.
